Europäisches Patentamt

European Patent Office

Office européen des brevets

(19)

(11) **EP 1 177 801 A1**

(12) **EUROPEAN PATENT APPLICATION**
published in accordance with Art. 158(3) EPC

(43) Date of publication:
**06.02.2002 Bulletin 2002/06**

(21) Application number: **01904520.2**

(22) Date of filing: **16.02.2001**

(51) Int Cl.⁷: **A61M 1/14**, A61K 33/14

(86) International application number:
**PCT/JP01/01137**

(87) International publication number:
**WO 01/60428 (23.08.2001 Gazette 2001/34)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **16.02.2000 JP 2000037969
03.03.2000 JP 2000058592**

(71) Applicant: **TEIJIN LIMITED
Osaka-shi Osaka 541-0054 (JP)**

(72) Inventors:
• **DEGUCHI, Tsuneo C/O TEIJIN Ltd
Tokyo Research Ctr
Hino-shi Tokyo 191-0065 (JP)**

• **IMAI, Ken C/O TEIJIN LTD, Tokyo Research Ctr
Hino-shi Tokyo 191-0065 (JP)**
• **SHIMURA, H. C/O TEIJIN LTD
Tokyo Research ctr
Hino-shi Tokyo 191-0065 (JP)**
• **MORIYAMA, Naohiko C/O TEIJIN LIMITED
Chiyoda-ku Tokyo 100-0011 (JP)**
• **ISHIHARA, Noriyuki
Amagasaki-shi, Hyogo 661-0965 (JP)**

(74) Representative: **HOFFMANN - EITLE
Patent- und Rechtsanwälte Arabellastrasse 4
81925 München (DE)**

(54) **DIALYZING FLUID PREPARING DEVICE AND POWDERY DIALYZING FLUID PREPARING CHEMICAL**

(57)     The invention provides a dialysate solution preparing apparatus for preparing a dialysate solution by mixing a powder dialysate medicament with water, in particular RO water. The dialysate solution preparing apparatus has a partition wall defining an accommodation chamber for accommodating a the container filled with the powder dialysate medicament. A container holder, provided in the accommodation chamber, holds the container at an inverted orientation where an opening of the container is disposed at the down side. A tank is provided under the holding means. After a cutter cuts the sealing member so that the powder dialysate medicament falls from the container, the water is applied to the powder dialysate medicament through a nozzle. The solution of the water and the powder dialysate medicament is supplied to the tank through a conduit. The dialysate solution preparing apparatus of the invention further has a circuit for directing the water or the solution in the tank to the nozzle and a heater provided in the circuit. According to a feature of the invention, the partition wall is composed of a thermal insulation material.

Fig.1

**EP 1 177 801 A1**

**Description**

TECHNICAL FIELD

The present invention relates to an apparatus for preparing a dialyzate solution for use in hemodialysis treatment, and in particular to an apparatus for preparing a dialyzate solution, which can effectively dissolve a powder dialysis medicament.

Further, the invention relates to a powder dialysis medicament for preparing a dialyzate solution, which medicament includes sodium chloride and sodium acid carbonate filled in a container. In particular, the invention relates to a sodium hydrogen carbonate based powder dialysis medicament filled in a container which medicament is improve to prevent solidification and caking of the powder during the storage thereof.

BACKGROUND ART

Hemodialysis treatment is a most effective treatment for renal insufficiency. In hemodialysis treatment, the blood and a dialyzate solution are supplied to a dialyzer. The dialyzer removes waste product and/or excess water from the blood through a hollow-fiber type semipermeable membrane module including a macromolecule membrane of cellulose, polysulfone or the like. The treated blood is returned to the patient. Acetic acid based medicament (A-type medicament) and bicarbonate based medicament (B-type medicament) are generally used for preparing a dialyzate solution. The A-type medicament is generally provided in the form of a enriched liquid including dextrose, sodium chloride, potassium chloride, calcium chloride, magnesium chloride and acetic acid. The B-type medicament is generally provided in the form of powder medicament including sodium acid carbonate and sodium chloride. In this connection, Japanese Unexamined Patent Publication (Kokai) No. 6-178802 discloses an A-type medicament in the form of powder.

Japanese Unexamined Patent Publication (Kokai) No. 9-618 discloses an apparatus for automatically preparing a dialyzate solution. The apparatus includes a cylindrical cutter (spike), having an inclined end, which is movable in the vertical direction for cutting a sealed opening of a container opposed to the cutter and a spray nozzle for spraying water into the container to wash the content out of the container.

Further, Japanese Unexamined Patent Publication (Kokai) No. 4-84967 describes an apparatus which supplies water in a bottle containing a powder medicament to wash it out into a tank with a stirrer for mixing the solution. A circulating pump promotes the mixing. Furthermore, Japanese Unexamined Patent Publication (Kokai) No. 5-168678 describes an apparatus in which a container for containing measured powder is held upside down so that the content falls by gravity into a tank which contains water. The tank includes an agitator for promotion of the mixing of the medicament with the water.

Pollution in a dialyzate solution preparing apparatus and the dialyzate solution supplying line causes proliferation of bacteria in the pips and fever in a patient due to endotoxin. Therefore, washing and sterilization of the apparatus and the dialyzate solution supplying line is required.

Above-mentioned Japanese Unexamined Patent Publication (Kokai) No. 9-618 discloses a method for sterilizing an artificial kidney. The method includes heating water up to the sterilization temperature and circulating the heated water through the water treatment module, an extracorporeal circuit and the dialyzate solution circuit. When an apparatus is washed and sterilized by circulating heated water through the apparatus, the washing and the sterilization is often carried out with a container, filled with a powder dialysis medicament to be used for preparing the next dialyzate solution, being mounted to the apparatus. However, if an apparatus is sterilized with a container filled with the medicament mounted, the powder dialysis medicament in the container is also heated so that the medicament in particular the B-type medicament is sometimes solidified and caked in the container. The dialyzate solution, which has been prepared by the solidified and caked B-type medicament, has no problem. However, the solidification and caking of the powder makes the removal of the powder dialysis medicament from the container difficult and the time needed to dissolve the powder dialysis medicament into the water longer.

Further, the B-type medicament including sodium acid carbonate and sodium chloride is free-flowing powder at the production time. However, during the storage under ambient temperature, the powder if sometimes solidified and caked in the container.

DISCLOSURE OF THE INVENTION

The present invention is directed to solve the prior art problems, and the objective of the invention is to provide an apparatus for preparing a dialyzate solution, which apparatus is improved to discharge substantially the whole amount of powder dialysis medicament from a container and to reduce the time for dissolving and further to facilitate washing and sterilization of the apparatus.

Further, the objective of the invention is to provide a powder dialysis medicament improved to prevent solidification and caking during the storage in the container.

The invention provides an apparatus for preparing a dialysate solution by mixing a powder dialysate medicament with water, in particular RO water. The powder dialysis medicament is filled in a container which includes a tubular main body portion defining a inside volume, a bottom wall formed at one end of the main body portion and a sealing member for sealingly closing an opening defined at the other end of the main body portion opposite to the bottom wall. The dialysate solution preparing apparatus has a partition wall defining a accommodation chamber for accommodating the container filled with the powder dialysate medicament. A container holder is provided in the accommodation chamber to hold the container in inverted orientation where the opening of the container is downwardly oriented. A cutter, for cutting the sealing member, is provided to move toward and away from the sealing member. A tank is disposed below the container holder. After the sealing member is cut by the cutter, water is applied through a nozzle to the powder dialysis medicament falling from the container. The solution of the water through the nozzle and the powder dialysis medicament is supplied to the tank through a conduit. The dialysate solution preparing apparatus further includes a circulation line for directing the water or solution in the tank to the nozzle. A heater, for heating the water in the circulation line, is provided in the circulation line. The invention is characterized by the partition wall comprising a thermal insulating material.

The dialyzate solution preparing apparatus of the invention prevents the heating of the powder dialysis medicament to prevent the solidification and caking of the powder dialysis medicament when washing and sterilization are executed by circulating heated water through the circulation line because the partition wall, defining the accommodation chamber for accommodating the container which is filled with the powder dialysate medicament, comprises a thermal insulation material.

The thermal insulating material is preferably includes foamed urethane. Further, cooling means may be provided for cooling the container accommodated in the accommodation chamber. The cooling means may include, for example a fan and a cooling air supplying conduit for directing the air to the container from the fan.

The container is preferably made of high density polyethylene, and the sealing member may comprises a heat sealable material having water permeability of or lower than $1g/(m^2 \cdot 24h)$. The powder dialysis medicament, filled in the container, is preferably the B-type medicament which includes sodium chloride and sodium acid carbonate.

Further , according to the invention, there is provided a powder dialysis medicament which is filled in a container including a tubular main body for defining an inside volume for receiving the powder dialysis medicament, a bottom wall defined at one end of the main body and a sealing member for closing an opening defined at the other end of the main body opposite to the bottom wall. The powder dialysis medicament comprises sodium chloride and sodium acid carbonate, the sodium chloride and sodium acid carbonate being filled in the container after they are mixed with each other.

The sodium chloride and the sodium acid carbonate include water of or lower than 0.5%(W). The sealing member comprises a heat sealable material having water permeability of or lower than $1g/(m^2 \cdot 24h)$.

BRIEF DESCRIPTION OF THE DRAWINGS

Figure 1 is a schematic diagram of a dialyzate solution preparing apparatus according a first embodiment of the invention.

Figure 2 is a schematic diagram of a dialyzate solution preparing apparatus according a second embodiment of the invention.

Figure 3 is a schematic section of a container filled with a powder dialysis medicament.

BEST MODE FOR CARRYING OUT THE INVENTION

With reference to the drawings, a preferred embodiment of the invention will be described hereinafter.

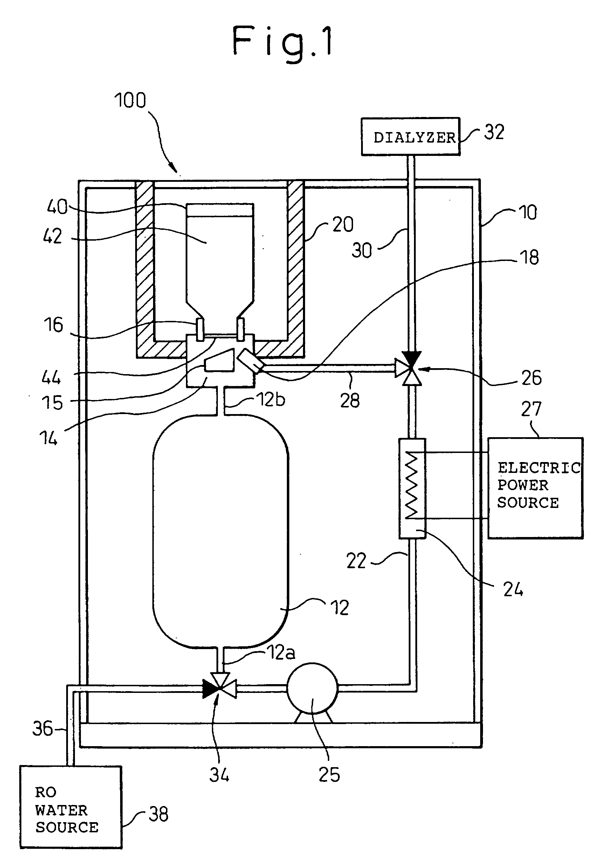

Referring to Figure 1, a dialyzate solution preparing apparatus 100 according to a first embodiment includes a tank 12 which is disposed in the inside volume 10a of a casing 10. The tank 12 is connected to a three way valve 34 through an outlet conduit 12a provided at the bottom of the tank 12. The tank 12 is further fluidly connected to a dissolving chamber 14 through an inlet conduit 12b provided at the top of the tank 12. Provided at the top of the dissolving chamber 14 is a container holder 16 which holds a container 40, filled with a powder dialysis medicament, in particular the B-type medicament 42, in inverted orientation. In the dissolving chamber 14, a cutter 15 is provided to move toward and away from a sealing member 44 of the container 40 held by the container holder 16. The cutter 15 is generally referred to a spike having a cylindrical body and a cutting edge provided at the end the body. The cutting edge is adapted to cut the sealing member 44 along the periphery of an opening 42c of the container 40. PCT/JP99/07165 discloses the detail of the cutter 15, which is incorporated by referred.

The three way valve 34 is connected to a RO water source 38 through a RO water supplying conduit 36. The three way valve 34 is further connected to a circulation conduit 22. The three way valve 34 may comprise a two-position type directional control valve and is movable between a first position at which the outlet passage 12b and the circulation conduit 22 are fluidly connected and a second position at which the outlet passage 12b and the RO water supplying conduit 36 are fluidly connected.

The RO water source 38 is a water treatment apparatus which removes minute particles, bacterium, ions or the like by passing the water under a high pressure through a reverse osmosis membrane to produce a RO water (a reverse osmosis water). A pump 25 for pumping the RO water and/or the solution contained in the tank 12 and a heater 24 for heating the RO water in the circulation conduit 22 are provided in the circulation conduit 22. The heater 24 may comprise an electric heater supplied with an electric power from an electric power source 27.

A directional control valve or three-way valve 26 is connected to the other end of the circulation conduit 22, The three-way valve 26 is connected to a nozzle 18, extending into the dissolving chamber 14, through a nozzle conduit 28. The valve is further fluidly connected to a dialyzer 32 through a dialyzate solution supplying conduit 30. The three-way valve 26 may comprise a two-position-type directional control valve, similar to the three way valve 34, which moves between a first position at which the circulation conduit 22 and the nozzle conduit 28 are fluidly connected to each other, and a second position at which the circulation conduit 22 and the dialyzate solution supplying conduit 30 are fluidly connected to each other.

In the present embodiment. the dialyzate solution preparing apparatus 100 further includes a partition wall 20 which defines, in the casing 10, an accommodation chamber for accommodating the container 40. The partition wall 20 comprises a thermal insulating material, preferably foamed urethane.

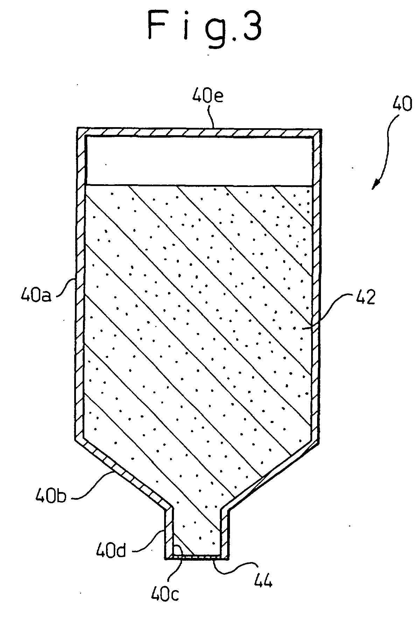

Referring to Figure 3, the container 40 comprises a tubular cylindrical main body portion 40a having a bottom wall 40e. At the other end of the main body portion 40a opposite to the bottom wall 40e, a neck 40d of a small diameter is provided through a tapered portion 40b. The neck 40d defines an opening 40c of the container 40. The opening 40c is sealingly closed by a sealing member 44 after the container 40 is filled with a powder dialysis medicament 42.

The operational function of the present embodiment will be described below.

When the dialyzate solution is prepared, first, the container 40, which is filled with the powder dialysis medicament 42, is held in inverted orientation, as shown in Figure 1. That is, the container 40 is mounted to the dialyzate solution preparing apparatus 100 by holding the neck 40d with the container holder 16 with the opening 40c being downwardly oriented. Then, a washing and sterilization operation is started with the container 40 mounted to the dialyzate solution preparing apparatus 100, as described below.

The three way valve 34 moves to the second position to fluidly connect the outlet passage 12b and the RO water supplying conduit 36 to each other so that the RO water is supplied to the tank 12 from the RO water source 38. Once the RO water is supplied to the tank 12 so that the water surface reaches a predetermined level, the three way valve 34 moves to the first position and the three-way valve 26 moves to the first position. This allows the outlet passage 12b of the tank 12 and the circulation conduit 22 to fluidly communicate with the circulation conduit 22 and with the nozzle conduit 28, respectively. Under this condition, activating the pump 25 directs the RO water in the tank 12 into the dissolving chamber 14 from the tank 12 through the outlet passage 12b, the circulation conduit 22 , the nozzle conduit 28 and the nozzle 18. The RO water directed into the dissolving chamber 14 is returned in the tank 12 through the inlet conduit 12b of the tank 12.

When the RO water is circulated in the circulation conduit 22, the heater 24 is activated to heat the RO water up to a sterilization temperature of 80-100 Celsius degrees, preferably 85-100 Celsius degrees. Circulating the heated RO water, as described above, washes and sterilizes the surface of the sealing member 44 attached to the opening of the container 40, inner surfaces of the dissolving chamber 14, the inlet conduit 12b, the tank 12 and the outlet passage 12b, the inside of the three way valve 34, the inner surface of the circulation conduit 22, the insides of the pump 25, the heater 24 and the three-way valve 26 and the inner surfaces of the nozzle 18 and the nozzle 18 with the high temperature RO water directly contacting therewith. Further, the inside of the casing 10 is also heated by the circulating RO water so that the temperature of the inside volume of the casing 10 increases to about 70 Celsius degrees.

In this way, in order ensure the sterilization of the dialyzate solution preparing apparatus 100, the above-describe high temperature RO water is circulated for a predetermined period, for example for at least an hour. In the prior art, the container 40 is exposed to the inside of the casing 10. Thus, in the sterilization process, the powder dialysis medicament, in particular the B-type medicament filled in the container 40, is heated so that the powder of sodium acid carbonate and sodium chloride is heated, which results in the problems of the solidification and caking. According to the present embodiment, the container 40 is disposed in the accommodation chamber defined by the partition wall 20 comprising the insulating material. Therefore, the heating of the powder of sodium acid carbonate and sodium chloride filled in the container, up to the temperature at which the solidification and caking of the powder occurs, during the sterilization process is prevented. In this connection, in order to prevent excessive heating of the container 40, the upper portion of the accommodation chamber is opened, as shown in Figure 1.

When the sterilization process is completed, the RO water in the tank 12 used for the sterilization process is discharged through a drain line (not shown) to the outside of the tank 12. Then, the three way valve 34 moves to the first position to supply the RO water again to the tank 12 from the RO water source 38. Once a predetermined amount of the RO water is held by the tank 12, the three way valve 34 moves to the second position. Then, the sealing member 44 is cut by a cutter for unsealing the container. This allows the powder medicament in the container 40 to fall by gravity into the dissolving chamber 14.

The pump 25 is activated to direct the RO water, held in the tank 12, into the opened container 40 through the nozzle 18. This washes the whole amount of the powder medicament out of the container 40 into the dissolving chamber 14. Further, in the dissolving chamber 14, the powder medicament is dissolved into the RO water directed through the nozzle 18 to flow out into the tank 12 through the inlet conduit 12b. In this way, circulating the RO water in the tank 12 for a predetermined period through a circulation line comprising the tank 12, the outlet passage 12b, the circulation conduit 22, the pump 25, the nozzle conduit 28, the nozzle 18, the dissolving chamber 14 and the inlet conduit 12b discharges the whole amount of the powder medicament in the container 40 into the tank 12 and mixes the medicament uniformly with the RO water. In order to promote the mixing, an agitator (not shown) may be provided in the tank 12.

Once the medicament is mixed uniformly with the RO water, the three-way valve 26 moves to the second position to supply the prepared dialyzate solution to the dialyzer 32.

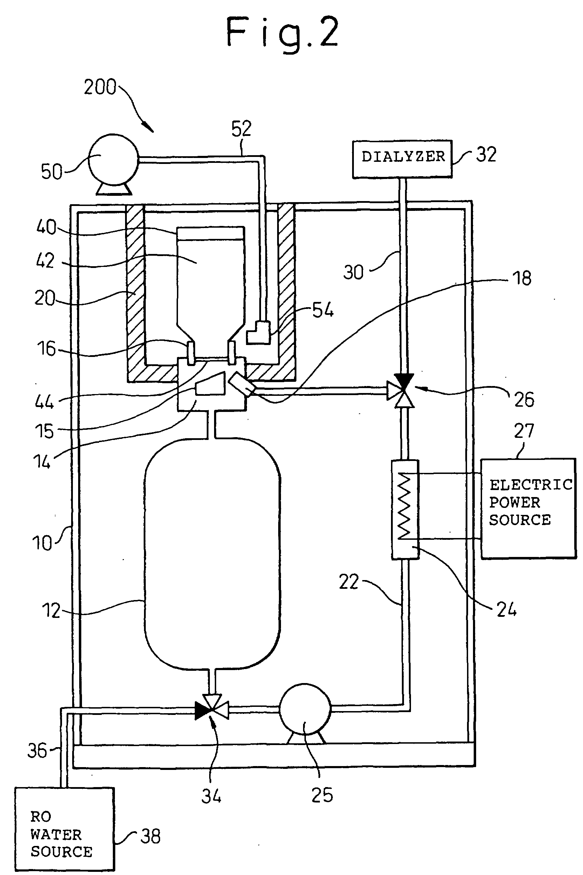

Referring to Figure 2, a second embodiment of the invention will be described.

The second embodiment is different from the first embodiment in the point of a feature which includes cooling means comprising a blower or a fan 50, a cooling air supplying conduit 52 and a cooling air nozzle 54. The remaining configuration is the same as the first embodiment, and the elements the same as the embodiment shown in Figure 1 are indicated by the same reference numbers.

Supplying a cooling air, during the sterilization process, by means of the blower or fan 50, the cooling air supplying conduit 52 and the cooling air nozzle 54 to the container in the accommodation chamber, at a predetermined flow rate, for example 20 liter/min ensures to prevent the container 40 to be heated. In order to effectively cool the container 40, the air nozzle 54 is preferably oriented to direct the cooling air to the container 40. Further, the cooling means is designed to keep the container 40 at a temperature lower than 50 Celsius degrees, more preferably lower than 40 Celsius degrees to prevent the solidification and caking of the powder medicament.

The cooling means may employ a circulation of cooling water or a Peltier device.

Referring to Figure 3, the container 40 is filled with the B-type medicament 42 including sodium acid carbonate and sodium chloride for preparing sodium hydrogencarbonate based dialyzate solution.

Conventionally, when a container is filled with a powder medicament, each of the components of a predetermined amount is sequentially filled in the bottle, and the opening is sealingly closed by a sealing member. According to the prior art, when the B-type medicament of powder dialysis medicament is filled in a bottle, sodium acid carbonate and sodium chloride are put separately sequentially in the bottle so that layers of sodium acid carbonate and sodium chloride are formed in the container. When the powder dialysis medicament thus produced is stored for a long period under the ambient temperature, the powder dialysis medicament receives a temperature history, which results in the solidification and caking thereof.

The inventors of this application found that filling sodium acid carbonate and sodium chloride into the container after they are mixed with each other prevents such solidification and caking. The mixing of sodium acid carbonate and sodium chloride can be carried out by a various mixing machines such as V-type mixer or a cross rotor mixer.

Water content of the powder dialysis medicament effects on the solidification and caking during the storage of the medicament so that the higher the water content the easier the solidification occurs. Sodium chloride with water content of or lower than 0.5% is preferably filled in the container 40. Likewise, sodium acid carbonate with water content of or lower than 0.5% is preferably used. In this connection, water content is defined by the following equation.

$$\gamma = w0/Wp$$

where

$\gamma$: water content (%)
W0: weight of water contained in the powder medicament
Wp: mass of the powder medicament before it is dried

The container 40 is formed of a material with low permeability suitable for medical use, preferably high density polyethylene(HDPE). The sealing member 40c is formed of a material which has low water permeability of or lower

than preferably 1g/(m$^2$•24h) to prevent the solidification and caking of the powder dialysis medicament 42. In this connection, the sealing member 40c was tested to determine its water permeability according to B method defined by JIS (Japan Industrial Standard) K7126.

Examples of the powder dialysis medicament according to the invention are shown in the following.

EXAMPLE 1

Sodium chloride (400g with water content of 0.05%) and sodium acid carbonate (200g with water content of 0.05%) were mixed with each other by a V-type mixing machine for 10 minutes under an ambient condition of 25 Celsius degrees and 15%RH (Relative Humidity). The mixture is filled in the container 40 formed of HDPE. The opening 40c of the container 40 is sealingly closed by a three-layer film (PET (polyethylene terephthalate) film/Silica coated PET film/PE (polyethylene) film) which has water permeability of 0.1g/(m$^2$•24h).

EXAMPLE 2

Sodium chloride (400g with water content of 0.05%) and sodium acid carbonate (200g with water content of 0.05%) were mixed with each other by a V-type mixing machine for 10 minutes under an ambient condition of 25 Celsius degrees and 50%RH. The mixture is filled in the container 40 formed of HDPE. The opening 40c of the container 40 is sealingly closed by the three-layer film (PET (polyethylene terephthalate) film/Silica coated PET film/PE(polyethylene) film) which has water permeability of 0.1g/(m$^2$•24h).

COMPARATIVE EXAMPLE 1

Sodium chloride (400g with water content of 0.05%) and sodium acid carbonate (200g with water content of 0.05%) were filled in the container 40 formed of HDPE sequentially in the order of sodium acid carbonate and sodium chloride under the condition of 25 Celsius degrees and 15%RH. The opening 40c of the container 40 is sealingly closed by a three-layer film (PET (polyethylene terephthalate) film/Silica coated PET film/PE(polyethylene) film) which has water permeability of 0.1g/(m$^2$•24h).

COMPARATIVE EXAMPLE 2

Sodium chloride (400g with water content of 0.05%) and sodium acid carbonate (200g with water content of 0.05%) were filled in the container 40 formed of HDPE sequentially in the order of sodium acid carbonate and sodium chloride under the condition of 25 Celsius degrees and 50%RH. The opening 40c of the container 40 is sealingly closed by a three-layer film (PET (polyethylene terephthalate) film/Silica coated PET film/PE(polyethylene) film) which has water permeability of 0.1g/(m$^2$•24h).

Each four containers, filled with powder dialysis medicament according to EXAMPLES 1 and 2 and COMPARATIVE EXAMPLES 1 and 2, were produced and stored for one month under an ambient condition in which temperature alternates within a range of 5 - 30 Celsius degrees. The powder in each of the containers was visually observed as to whether the solidification and caking of the powder in the bottle occurs or not. Further, after the one-month storage, percentage of discharge of the content through the opening of each of the containers was determined by holding the containers in the inverted orientation and cutting the sealing member so that the content falls by gravity. The percentage of discharge was obtained by holding the container in the inverted orientation and measuring the changes in weight between before and after the sealing member was cut and basis on the weight of the empty container. The percentage of discharge is indicated by average of each of the four containers. The results are indicated in TABLE 1.

TABLE 1

| | NUMBER OF BOTTLES SOLIDIFICATION WAS OBSERVED | FIRST TIME SOLIDIFICATION WAS OBSERVED | PERCENTAGE OF DISCHARGE |
|---|---|---|---|
| EXAMPLE 1 | 0 | - | 99.8% |
| EXAMPLE 2 | 0 | - | 99.9% |
| COMPARATIVE EXAMPLE 1 | 4 | 5 days | 70.7% |

TABLE 1   (continued)

|  | NUMBER OF BOTTLES SOLIDIFICATION WAS OBSERVED | FIRST TIME SOLIDIFICATION WAS OBSERVED | PERCENTAGE OF DISCHARGE |
|---|---|---|---|
| COMPARATIVE EXAMPLE 2 | 4 | 3 days | 65.7% |

As shown in TABLE 1, in COMPARATIVE EXAMPLES 1 and 2, the solidification and caking of the powder dialysis medicament was observed in all of the containers within three to five days from the start of the experiment. On the other hand, in EXAMPLES 1 and 2, the solidification and caking of the powder dialysis medicament was not observed in all of the containers even after one month from the start of the experiment. Further, the percentage of discharge was about 70% in COMPARATIVE EXAMPLES 1 and 2. On the other hand, in Examples 1 and 2, percentage of discharge of at least 99.8% was obtained so that substantially the whole amount of the powder dialysis medicament filled in the container 40 was discharged.

**Claims**

1.  An apparatus for preparing a dialyzate solution, comprising:

    a partition wall defining an accommodation chamber for accommodating a container which includes a tubular main body for defining an inside volume for receiving a powder dialysis medicament, a bottom wall defined at one end of the main body and a sealing member for closing an opening defined at the other end of the main body opposite to the bottom wall;
    a container holder for holding the container, within the accommodation chamber, in an inverted orientation where the opening is downwardly oriented;
    a cutter, provided to move toward and away from the sealing member, for cutting the sealing member;
    a tank disposed below the container holder;
    a nozzle for applying water to the powder dialysis medicament falling from the container after the sealing member is cut by the cutter;
    a conduit for directing the solution which is a mixture of the water, supplied through the nozzle, and the powder dialysis medicament;
    a circulation line for directing the water and/or the solution in the tank to the nozzle;
    a heater provided in the circulation line for heating the water in the circulation line; and
    the partition wall comprising a thermal insulating material.

2.  An apparatus according to claim 1 wherein the thermal insulating material includes foamed urethane.

3.  An apparatus according to claim 2, further comprising a cooling means for cooling the container held in the accommodation chamber.

4.  An apparatus according to claim 3 wherein the cooling means comprises a fan, a cooling air supplying conduit for directing air to the container from the fan.

5.  An apparatus according to claim 1 wherein the container is made of high density polyethylene; and
    the sealing member comprising a heat sealable material having water permeability of or lower than 1g/$(m^2 \cdot 24h)$.

6.  An apparatus according to claim 1 wherein the powder dialysis medicament, filled in the container, includes sodium chloride and sodium acid carbonate.

7.  An apparatus according to claim 6, wherein the sodium chloride and sodium acid carbonate are filled in the container after they are mixed with each other.

8.  An apparatus according to claim 7, wherein the sodium chloride and the sodium acid carbonate include water of or lower than 0.5%(W).

9. A powder dialysis medicament adapted to use in an apparatus for preparing a dialyzate solution which apparatus comprises:

a partition wall defining an accommodation chamber for accommodating a container which includes a tubular main body for defining an inside volume for receiving the powder dialysis medicament, a bottom wall defined at one end of the main body and a sealing member for closing an opening defined at the other end of the main body opposite to the bottom wall;

a container holder for holding the container, within the accommodation chamber, in an inverted orientation where the opening is downwardly oriented;

a cutter, provided to move toward and away from the sealing member, for cutting the sealing member;

a tank disposed below the container holder;

a nozzle for applying water to the powder dialysis medicament falling from the container after the sealing member is cut by the cutter; and

a conduit for directing the solution of the water, supplied through the nozzle, and the powder dialysis medicament;

the powder dialysis medicament comprising sodium chloride and sodium acid carbonate, the sodium chloride and sodium acid carbonate being filled in the container after they are mixed with each other.

10. A powder dialysis medicament according to claim 9, wherein the sodium chloride and the sodium acid carbonate include water of or lower than 0.5%(W).

11. A powder dialysis medicament according to claim 9, wherein the container is made of high density polyethylene; and the sealing member comprising a heat sealable material having water permeability of or lower than $1g/(m^2 \cdot 24h)$.

12. A powder dialysis medicament which is filled in a container including a tubular main body for defining an inside volume for receiving the powder dialysis medicament, a bottom wall defined at one end of the main body and a sealing member for closing an opening defined at the other end of the main body opposite to the bottom wall,

the powder dialysis medicament comprising sodium chloride and sodium acid carbonate, the sodium chloride and sodium acid carbonate being filled in the container after they are mixed with each other.

13. A powder dialysis medicament according to claim 12, wherein the sodium chloride and the sodium acid carbonate include water of or lower than 0.5%(W).

14. A powder dialysis medicament according to claim 12, wherein the container is made of high density polyethylene; and the sealing member comprises a heat sealable material having water permeability of or lower than $1g/(m^2 \cdot 24h)$.

# Fig.1

# Fig.2

# Fig.3

## INTERNATIONAL SEARCH REPORT

| | International application No. |
| --- | --- |
| | PCT/JP01/01137 |

| A. CLASSIFICATION OF SUBJECT MATTER |
| --- |
| Int.Cl$^7$  A61M1/14, A61K33/14 |
| According to International Patent Classification (IPC) or to both national classification and IPC |

| B. FIELDS SEARCHED |
| --- |
| Minimum documentation searched (classification system followed by classification symbols) |
| Int.Cl$^7$  A61M1/14, A61K33/14 |
| Documentation searched other than minimum documentation to the extent that such documents are included in the fields searched |
| Jitsuyo Shinan Koho        1926-1996    Toroku Jitsuyo Shinan Koho  1994-2001<br>Kokai Jitsuyo Shinan Koho   1971-2001    Jitsuyo Shinan Toroku Koho  1996-2001 |
| Electronic data base consulted during the international search (name of data base and, where practicable, search terms used) |

| C. DOCUMENTS CONSIDERED TO BE RELEVANT |

| Category* | Citation of document, with indication, where appropriate, of the relevant passages | Relevant to claim No. |
| --- | --- | --- |
| A<br>Y | EP, 469487, A1 (Nikkiso Co., Ltd.),<br>05 February, 1992 (05.02.92),<br>Full text<br>& US, 5547645, A1   & JP, 4-84967, A | 1-8<br>9-14 |
| A<br>Y | WO, 96/25214, A1 (AKSYS, Ltd.),<br>22 August, 1996 (22.08.96),<br>Full text<br>& US, 5591344, A1   & JP, 9-618, A | 1-8<br>9-14 |
| Y | JP, 9-40562, A (Tomita Seiyaku K.K.),<br>10 February, 1997 (10.02.97),<br>Full text   (Family: none) | 6-14 |
| Y | JP, 10-87478, A (Tomita Seiyaku K.K.),<br>07 April, 1998 (07.04.98),<br>Full text   (Family: none) | 6-14 |
| A | EP, 697220, A1 (Fresenium AG),<br>21 February, 1996 (21.02.96),<br>Full text<br>& US, 5616305, A1   & JP, 8-168522, A | 1-14 |

☒ Further documents are listed in the continuation of Box C.     ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    15 May, 2001 (15.05.01) | Date of mailing of the international search report<br>    29 May, 2001 (29.05.01) |
| --- | --- |
| Name and mailing address of the ISA/<br>    Japanese Patent Office | Authorized officer |
| Facsimile No. | Telephone No. |

Form PCT/ISA/210 (second sheet) (July 1992)

12

**INTERNATIONAL SEARCH REPORT**

| International application No. |
| --- |
| PCT/JP01/01137 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP, 5-168678, A (Nikkiso Co., Ltd.), 02 July, 1993 (02.07.93), Full text   (Family: none) | 1-14 |

Form PCT/ISA/210 (continuation of second sheet) (July 1992)